# EUROPÄISCHE PATENTSCHRIFT

(11) **EP 1 319 011 B1**
(45) Veröffentlichungstag und Bekanntmachung des Hinweises auf die Patenterteilung: **05.12.2012**
(21) Anmeldenummer: 01972077.0
(22) Anmeldetag: 21.09.2001
(51) Int. Cl.: C07D 519/00, A61K 31/429, A61P 35/00

(54) **TRIAZOLO-EPOTHILONE**
TRIAZOLO-EPOTHILONES
TRIAZOLO-EPOTHILONES

(30) Priorität: 22.09.2000 DE 10047529; 27.02.2001 DE 10109426
(43) Veröffentlichungstag der Anmeldung: 18.06.2003
(73) Patentinhaber: Helmholtz-Zentrum für Infektionsforschung GmbH, 38124 Braunschweig (DE)
(72) Erfinder: HÖFLE, Gerhard, 38124 Braunschweig (DE); GLASER, Nicole, 38124 Braunschweig (DE)
(74) Vertreter: Boeters, Hans Dietrich
(86) Internationale Anmeldenummer: PCT/EP2001/010991
(87) Internationale Veröffentlichungsnummer: WO 2002/024712

(56) Entgegenhaltungen:
- EP-A- 0 625 520
- WO-A-00/50423
- HÖFLE G ET AL: "N-Oxidation of Epothilone A. C-and O-Acyl Rearrangement to C-19 and C-21 Substituted Epothilones" ANGEWANDTE CHEMIE. INTERNATIONAL EDITION, VERLAG CHEMIE. WEINHEIM, DE, Bd. 38, Juli 1999 (1999-07), Seiten 1971-4, XP002140116 ISSN: 0570-0833

## Beschreibung

Die Erfindung betrifft Triazolo-thiazol-Analoge von Epothilon A und Epothilon B.

Epothilone sind macrocyclische Lactone mit fungizider und cytotoxischer Wirkung. Es besteht ein kontinuierlicher Bedarf für Analoge oder Derivate mit vergleichbarer oder besserer Wirksamkeit, die als Fungizide oder Cytostatika verwendet werden können.

Eine Übersicht über die Chemie der Epothilone wird beispielsweise von Nicolaou et al. in Angew. Chem. Int. Ed. 1998, Bd. 37, 2014-2045 gegeben.

Aufgabe der Erfindung ist die Bereitstellung derartiger Epothilon-Analogen bzw. -Derivate.

Die Erfindung betrifft somit Triazolo-thiazol-Analoge von Epothilon A und Epothilon B mit der Formel 4a oder 4b: 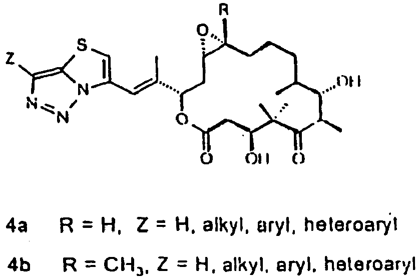 in denen bedeuten:
R: H, CH₃
Z: H, Alkyl, Aryl, Heteroaryl,
wobei bedeutet:
Alkyl: Methyl, Ethyl, Propyl, iso-Propyl, Butyl, iso-Butyl, tert.-Butyl, Pentyl oder Hexyl, gegebenenfalls substituiert mit C₁-C₆-Alkoxy, C₁-C₆-Acyl, Hydroxyl oder Halogen, insbesondere Br, Cl, F oder J, und/oder
Aryl: Phenyl, o-, m-, p-Tolyl, o-, m-, p-Xylyl, Benzyl, Phenethyl oder Naphthyl, gegebenenfalls substituiert mit C₁-C₆-Alkyl, C₁-C₆-Alkoxy, C₁-C₆-Acyl, Hydroxyl oder Halogen, insbesondere Br, Cl, F oder J, und/oder
Heteroaryl: Furanyl, Pyranyl, Pyrrolyl, Imidazolyl, Pyrazolyl, Pyridinyl oder Indolyl, gegebenenfalls substituiert mit C₁-C₆-Alkyl, C₁-C₆-Alkoxy, C₁-C₆-Acyl, Hydroxyl oder Halogen, insbesondere Br, Cl, F oder J.

Die erfindungsgemäßen Triazolo-thiazol-Epothilone sind sehr wirksame Fungizide und hochpotente Cytostatika mit günstigen pharmakologischen Eigenschaften.

Alkyl bedeutet geradkettiges oder verzweigtes C₁-C₆-Alkyl, das beliebig substituiert (einfach oder mehrfach) sein kann, beispielsweise Methyl, Ethyl, Propyl, iso-Propyl, Butyl, iso-Butyl, tert.-Butyl, Pentyl, Hexyl. Beispiele für Substituenten sind C₁-C₆-Alkoxy, C₁-C₆-Acyl, Hydroxyl, Halogen wie Brom, Chlor, Fluor und Jod.

Aryl bedeutet einkernige oder mehrkernige aromatische Systeme, die beliebig substituiert (einfach oder mehrfach) sein können, beispielsweise Phenyl, o-, m-, p-Tolyl, o-, m-, p-Xylyl, Benzyl, Phenethyl, Naphthyl. Beispiele für Substituenten sind C₁-C₆-Alkyl, C₁-C₆-Alkoxy, C₁-C₆-Acyl, Hydroxyl, Halogen wie Brom, Chlor, Fluor und Jod.

Heteroaryl bedeutet einkernige oder mehrkernige heteroaromatische Systeme, die beliebig substituiert (einfach oder mehrfach) sein können, wobei der aromatische Kern ein oder mehrere unter N, O, S ausgewählte Heteroatome aufweisen kann. Beispiel für Heteroaryl sind Furanyl, Pyranyl, Pyrrolyl, Imidazolyl, Pyrazolyl, Pyridinyl, Indolyl. Beispiele für Substituenten sind C₁-C₆-Alkyl, C₁-C₆-Alkoxy, C₁-C₆-Acyl, Hydroxyl, Halogen wie Brom, Chlor, Fluor und Jod.

Die Erfindung betrifft ferner ein Verfahren zur Herstellung der erfindungsgemäßen Triazolo-thiazol-Analogen von Epothilon A und Epothilon B sowie fungizide und pharmazeutische Zusammensetzungen, die ein oder mehrere derartige Analoge enthalten, und die Verwendung der Analogen und der diese enthaltenden fungiziden oder pharmazeutischen Zusammensetzungen zur Bekämfung von Pilzen oder zur Behandlung von Erkrankungen, die sich mit Cytostatika behandeln lassen, beispielsweise Tumorerkrankungen wie Krebs oder zellwachstumsstörungen.

Die fungiziden und pharmazeutischen Zusammensetzungen können neben dem eigentlichen Wirkstoff übliche Träger, Verdünnungsmittel oder Hilfsstoffe enthalten, beispielsweise Stabilisatoren wie UV-Absorber, Antioxidationsmittel, Konservierungsmittel.

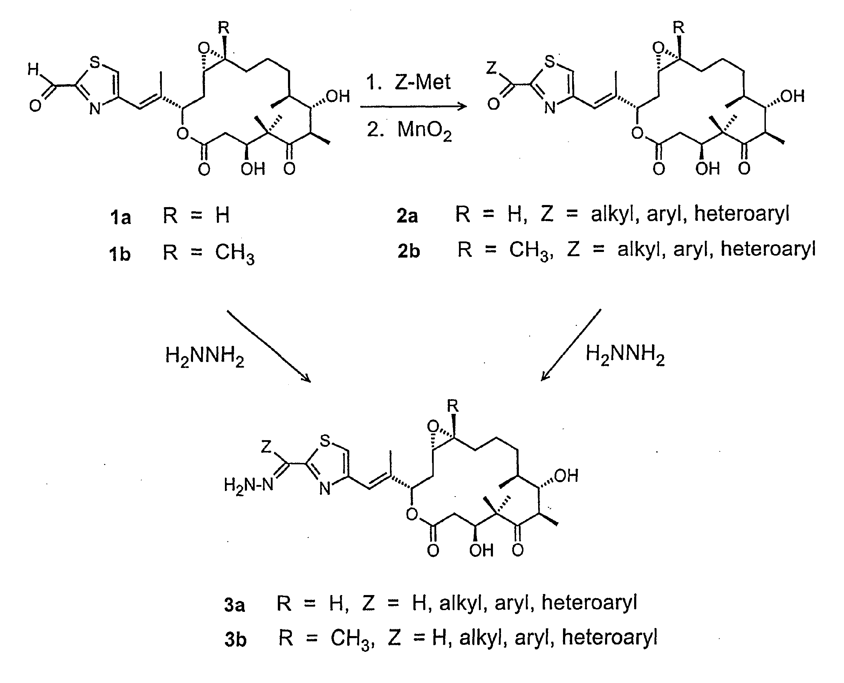

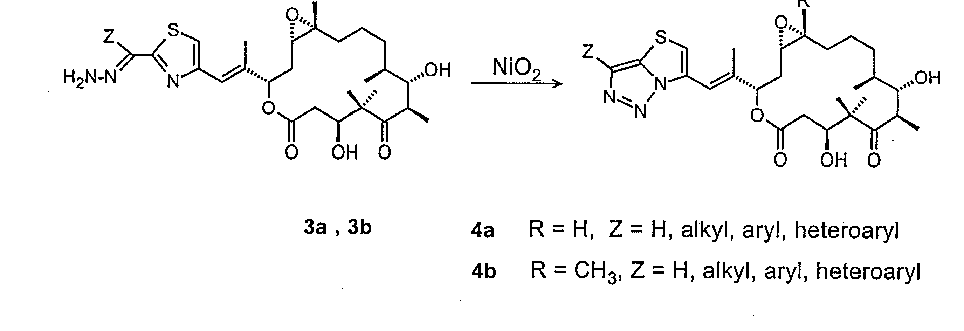

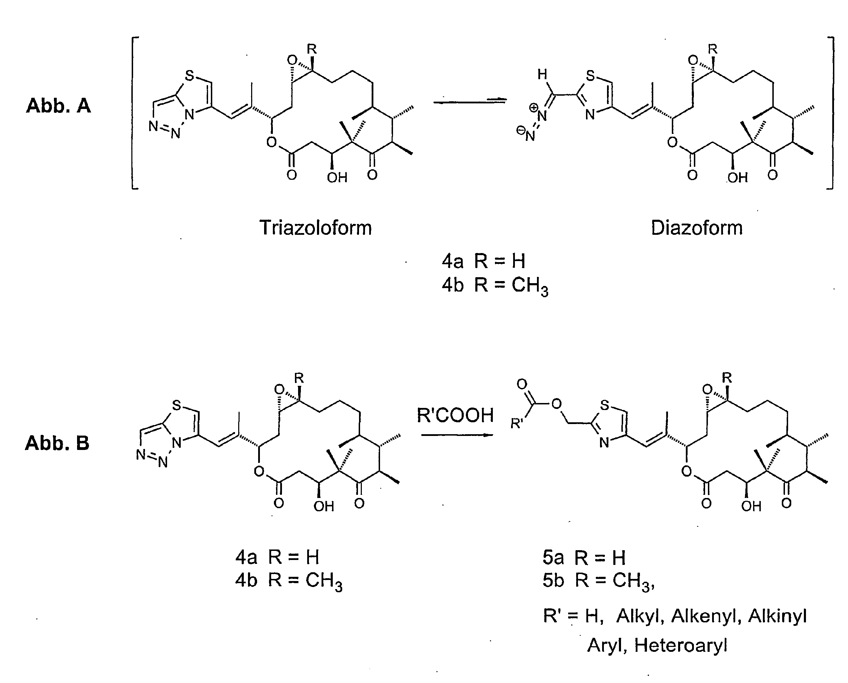

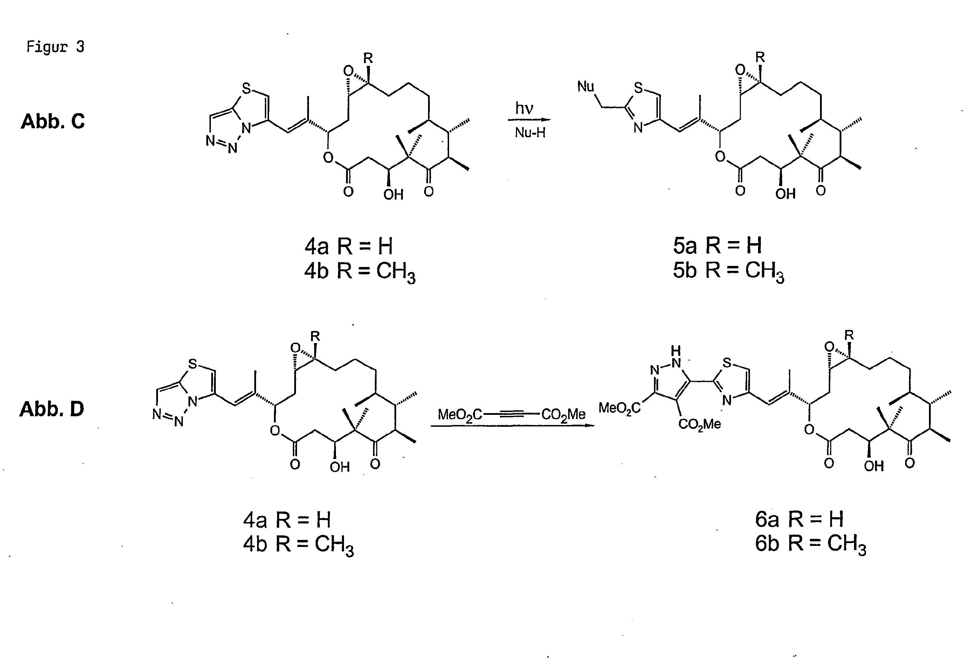

Figur 1 zeigt einen Syntheseweg zur Herstellung der erfindungsgemäßen Triazolo-thiazol-Analogen von Epothilon A und Epothilon B.

Im folgenden wird ohne Beschränkung unter Bezugnahme auf Figur 1 die Herstellung der erfindungsgemäßen Triazolo-thiazol-Analogen von Epothilon A und Epothilon B beschrieben. Die Abkürzung Met bedeutet Metall.

Die Herstellung von C21-modifizierten Epothilonen, d.h. der Aldehyde, Ketone und Hydrazone mit den Formeln 1, 2 bzw. 3, ist in der offengelegten deutschen Patentanmeldung DE 199 07 588 und in der offengelegten internationalen Patentanmeldung WO 2000/050423 der Anmelderin beschrieben. Bei dem erfindungsgemäßen Verfahren wird an den Hydrazon-Derivaten der Formeln 3a und 3b der oxidative Ringschluß mit Hilfe von Metalloxiden, vorzugsweise mit NiO₂, K₃[Fe(CN)₆], Bleitetraacetat oder Natriumhypochlorid (vgl. Houben-Weyl, Bd. E 14b, 4. Auflage, 1999) vorgenommen.

Synthese eines Triazolo-thiazol-Analogen von Epothilon A (Formel 4a, Z = H):

24.6 mg (47.2 µmol) Epothilon-A-21-aldehydhydrazon (Formel 3a) werden in 1.5 ml abs. Dichlormethan gelöst. Im Abstand von 15 min werden dreimal je 42.8 mg (472.2 µmol) Nickelperoxid hinzugegeben, wobei bei Raumtemperatur gerührt wird. Anschließend wird über Celite filtriert und mit Dichlormethan nachgewaschen. Die vereinigten organischen Phasen werden eingeengt und im Hochvakuum getrocknet. Die Reinigung des Rohproduktes erfolgt mittels präparativer HPLC (Laufmittel: Acetonitril / Wasser 38:62; Säule: Nucleosil 100 C18 7µm, 21x250 mm). Es wurden 12.0 mg (49 %) Produkt erhalten.

Die spektroskopischen Daten sind identisch mit Epothilon A (vgl. DE 4138042 C2) mit Ausnahme von:
¹H-NMR (400 MHz, CDCl₃): d = 2.25 (dt, 2a-H), 2.57 (dd, 2b-H), 4.63 (m, 3-H), 5.02 (dd, 3-OH), 1.68 (m, 14a-H), 2.31 (dt, 14b-H), 5.53 (d, 15-H), 6.92 (bs, 17-H), 7.06 (s, 19-H), 7.84 (s, 21-H), 1.08 (s, 22-H), 1.55 (s, 23-H); ¹³C-NMR (100 MHz, CDCl₃): d = 73.0 (3-C), 54.7 (4-C), 41.4 (6-C), 71.4 (7-C), 32.0 (14-C), 74.9 (15-C), 145.1 (16-C), 109.2 (17-C), 129.2 (18-C), 115.5 (19-C), 136.4 (20-C), 124.8 (21-C), 15.9 (C-22), 23.3 (23-C), 12.7 (24-.C); 18.1 (27-C); HRMS (DCI): C₂₆H₃₇N₃0₆S: [M+NH₄+] ber. 537.2747, gef. 537.2721.

Synthese eines Triazolothiazol-Analogen von Epothilon B (Formel 4b, Z = H):
8.1 mg (15.1 µmol) Epothilon-B-21-aldehydhydrazon (Formel 3b) werden in 1 ml abs. Dichlormethan gelöst. Zur Lösung werden 13.7 mg (151.4 µmol) Nickelperoxid gegeben, worauf 15 min bei Raumtemperatur gerührt wird. Das Nickelperoxid wird über Celite abfiltriert und mit Dichlormethan gewaschen. Die vereinigten organischen Phasen werden eingeengt und im Hochvakuum getrocknet. Die Reinigung des Rohproduktes erfolgt mittels präparativer HPLC (Laufmittel: Acetonitril / Wasser 40:60; Säule: Nucleosil 100 C18 7 µm, 21x250 mm) und ergab 4.7 mg (58 %) Produkt.

Die spektroskopischen Daten sind identisch mit Epothilon B (vgl. DE 4138042 C2) mit Ausnahme von:
¹H-NMR (400 MHz, CDCl₃): d = 2.25 (dt, 2a-H), 2.59 (dd, 2b-H), 4.69 (m, 3-H), 5.02 (dd, 3-OH), 1.76 (m, 14a-H), 2.31 (dt, 14b-H), 5.53 (d, 15-H), 6.91 (bs, 17-H), 7.06 (s, 19-H), 7.85 (s, 21-H), 1.08 (s, 22-H), 1.56 (s, 23-H); ¹³C-NMR (100 MHz, CDCl₃): d = 71.3 (3-C), 54.9 (4-C), 41.0 (6-C), 72.4 (7-C), 33.1 (14-C), 75.2 (15-C), 145.3 (16-C), 108.9 (17-C), 129.2 (18-C), 115.5 (19-C), 136.5 (20-C), 124.9 (21-C), 15.7 (22-C), 23.2 (23-C), 12.0 (24-C), 18.1 (27-C); MS (ESI): [M+H⁺] =534.

Die pharmakologisch Wirksamkeit ist in der folgenden Tabelle dargestellt:

### Wachstumstests mit Säugerzellkulturen

| Zell-Linie | Herkunft | Triazolo-Derivat von | |
|---|---|---|---|
| | | Epothilon A | Epothilon B |
| | | IC-50 [ng/ml] | |
| L929 | Maus (Unterhautfettgewebe) | 10 | 1,0 |
| K-562 | Mensch (Leukämie) | 6 | 0,7 |
| U-937 | Mensch (Lymphom) | 4 | 0,5 |

### Herstellung von 21-O-Acetyl-epothilon E (5a, R¹ = CH₃):

Zu einer Lösung von 3.2 mg (6.2 mmol) Triazolo-epothilon A in 250 ml Dichlormethan werden 2 ml (35.0 mmol) Eisessig gegeben und über Nacht bei Raumtemperatur gerührt. Der Reaktionsansatz wird mit Wasser versetzt und drei mal mit Essigester extrahiert. Die vereinigten organischen Phasen werden eingeengt und am Hochvakuum getrocknet. Es wurden 2.8 mg (82 %) 21-O-Acetyl-epothilon E erhalten.

Die spektroskopischen Daten sind identisch mit Epothilon A (vgl. DE 4138042 C2) mit Ausnahme von:
¹H-NMR (400 MHz, CDCl₃) : δ = 6.60 (bs, 17-H), 7.14 (s, 19-H), 5.34 (s, 21-H₂), 2.14 (s, 2' -H3) ; ¹³C-NMR (100 MHz, CDCl₃) : 137. 8 (C-16), 119.6 (C-17), 152.5 (C-18), 118.0 (C-19), 163.7 (C-20), 62.5 (C-21), 170.2 (C-1') , 20.9 (C-2') ; R_{f} (CH₂Cl₂/MeOH 95/5) : 0.45; HRMS (EI) : C₂₈H₄₁NO₈S: [M]⁺ ber. 551.2553, gef. 551.2519

Herstellung von Epothilon E-21-O-(3'-Methoxycarbonyl)-propin-säureester (5a, R' = CH₃OOC-C₂):
Zu einer Lösung von 5.1 mg (9.8 mmol) Triazolo-epothilon A in 400 ml Dichlormethan werden 7.0 mg (55.0 mmol) Acetylendicarbonsäuremonomethylester gegeben. Der Reaktionsansatz wird über Nacht bei Raumtemperatur gerührt, anschließend mit Wasser versetzt und drei mal mit Essigester extrahiert. Die vereinigten organischen Phasen werden eingeengt und am Hochvakuum getrocknet. Nach Reinigung mittels PSC (CH₂Cl₂/Methanol 95/5) wurden 3.6 mg (59 %) Epothilon E-21-O-(3'-Methoxycarbonyl)-propinsäureester erhalten.

Die spektroskopischen Daten sind identisch mit Epothilon A (vgl. DE 4138042 C2) mit Ausnahme von:
¹H-NMR (400 MHz, CDCl₃) : δ = 5.45 (dd, 15-H), 6.60 (bs, 17-H), 7.20 (s, 19-H), 5.49 (bs, 21-H₂), 3.85 (s, 5'-H₃) ; ¹³C-NMR (100 MHz, CDCl₃): 76.5 (C-15), 138.2 (C-16), 119.5 (C-17), 153.0 (C-18) , 118.8 (C-19) , 161.2 (C-20), 64.2 (C-21), 151.1 (C-1') , 152.0 (C-4'), 53.6 (C-5'); R_{f} (CH₂Cl₂/MeOH 95/5) : 0.32; MS (DCI) : [M+NH₄]⁺ = 637.

Photolyse von Triazolo-epothilon A zu 21-O-Methyl-epothilon A (5a, Nu-H = CH₃OH) :
9.7 mg (18.7 mmol) Triazolo-epothilon A werden in 1 ml Methanol gelöst und unter Kühlung (Eisbad 0°C) mit Hilfe einer Quecksilberdampflampe (Fa. DEMA, HPK-125) vier Stunden belichtet. Anschließend wird das Lösungsmittel reduziert und der Reaktionsansatz über präparative HPLC (CH₃CN/H₂O 40/60) aufgetrennt. Es wurden 2.1 mg (24 %) 21-Methoxy-epothilon A isoliert.

Die spektroskopischen Daten sind identisch mit Epothilon A (vgl. DE 4138042 C2) mit Ausnahme von:
¹H-NMR (300 MHz, CDCl₃): δ = 6.61 (bs, 17-H), 7.13 (s, 19-H), 4.71 (s, 21-H₂), 3.49 (s, 1'-H₃) ; ¹³C-NMR (75 MHz, CDCl₃) : 137.5 (C-16), 120.0 (C-17), 152.2 (C-18), 117.3 (C-19), 167.8 (C-20), 71.5 (C-21), 59.1 (C-1'); R_{f} (CH₂Cl₂/MeOH 95/5): 0.33; HRMS (EI): C₂₇H₄₁NO₇S: [M]⁺ ber. 523.2604, gef. 523.2609.

1,3-dipolare Cycloaddition von Acetylendicarbonsäuredimethylester und Triazolo-epothilon A zum Pyrazolderivat 6a:
1.8 mg (3.5 mmol) Triazolo-epothilon A werden in 200 ml Dichlormethan gelöst. Innerhalb von vier Stunden wird drei mal jeweils 4.3 ml (34.7 mmol) Acetylendicarbonsäure-dimethylester zugegeben und bei Raumtemperatur gerührt. Der Reaktionsansatz wird etwas eingeengt und über PSC (CH₂Cl₂/Methanol 95/5) aufgetrennt. Es wurden 2.0 mg (87 %) Cycloadditionsprodukt erhalten.

Die spektroskopischen Daten sind identisch mit Epothilon A (vgl. DE 4138042 C2) mit Ausnahme von:
¹H-NMR (400 MHz, CDCl₃) : δ = 5.49 (dd, 15-H), 6.63 (bs, 17-H), 7.29 (s, 19-H), 3.97 (s, 4'-H₃), 3.95 (s, 6'-H₃); R_{f} (CH₂Cl₂/MeOH 95/5) : 0.17; HRMS (DCI): C₃₂H₄₃N₃O₁₀S: [M+H]⁺ ber. 662.2742, gef. 662.2778.

## Patentansprüche

1. Triazolo-thiazol-Analoge von Epothilon A und Epothilon B mit der Formel 4a oder 4b: 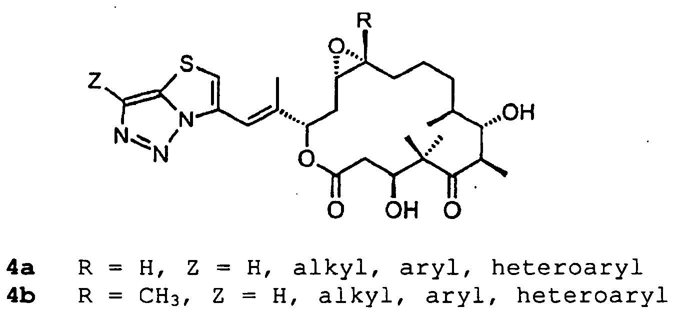 in denen bedeuten:
R: H oder CH₃
Z: H, Alkyl, Aryl oder Heteroaryl,
wobei bedeutet:
Alkyl: Methyl, Ethyl, Propyl, iso-Propyl, Butyl, iso-Butyl, tert.-Butyl, Pentyl oder Hexyl, gegebenenfalls substituiert mit C₁-C₆-Alkoxy, C₁-C₆-Acyl, Hydroxyl oder Halogen, insbesondere Br, Cl, F oder J, und/oder
Aryl: Phenyl, o-, m-, p-Tolyl, o-, m-, p-Xylyl, Benzyl, Phenethyl oder Naphthyl, gegebenenfalls substituiert mit C₁-C₆-Alkyl, C₁-C₆-Alkoxy, C₁-C₆-Acyl, Hydroxyl oder Halogen, insbesondere Br, Cl, F oder J, und/oder
Heteroaryl: Furanyl, Pyranyl, Pyrrolyl, Imidazolyl, Pyrazolyl, Pyridinyl oder Indolyl, gegebenenfalls substituiert mit C₁-C₆-Alkyl, C₁-C₆-Alkoxy, C₁-C₆-Acyl, Hydroxyl oder Halogen, insbesondere Br, Cl, F oder J.

2. Verfahren zur Herstellung der Triazolo-thiazol-Analogen von Epothilon A oder Epothilon B nach Anspruch 1, bei dem an einem Hydrazon-Derivat von Epothilon A oder Epothilon B mit der Formel 3a oder 3b: 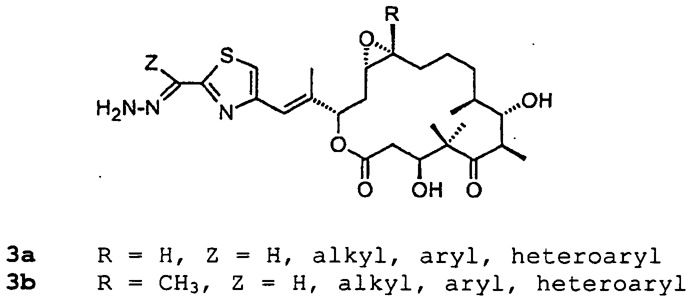 wobei R und Z die oben definierten Bedeutungen haben, mit Hilfe von Metalloxiden, K₃[Fe(CN)₆], Bleitetraacetat oder Natriumhypochlorid ein oxidativer Ringschluß vorgenommen wird.

3. Verfahren nach Anspruch 2, wobei das Metalloxid Ni0₂ ist.

4. Pharmazeutische Zusammensetzung, enthaltend oder bestehend aus einem oder mehreren Triazolo-thiazol-Analogen von Epothilon A oder Epothilon B nach Anspruch 1 neben einem pharmazeutisch akzeptablen Träger, Verdünnungsmittel oder Hilfsstoff.

5. Triazolo-thiazol-Analogon von Epothilon A oder Epothilon B nach Anspruch 1 oder einer pharmazeutischen Zusammensetzung nach Anspruch 4 zur Verwendung bei der Behandlung von Tumorerkrankungen und Zellwachstumsstörungen.

6. Triazolo-thiazol-Analogon von Epothilon A oder Epothilon B nach Anspruch 1 oder einer pharmazeutischen Zusammensetzung nach Anspruch 4 zur Verwendung wie in Anspruch 5, wobei es sich bei der Tumorerkrankung um Krebs handelt.

7. Fungizide Zusammensetzung, enthaltend oder bestehend aus einem oder mehreren Triazolo-thiazol-Analogen von Epothilon A oder Epothilon B nach Anspruch 1 neben einem für Fungizide akzeptablen Träger, Verdünnungsmittel oder Hilfsstoff.

8. Verfahren zur Herstellung von C-21-Estern von Epothilon A mit der Formel 5a und/oder Epothilon B mit der Formel 5b, bei dem man Triazolo-epothilon A gemäß Formel 4a von Anspruch 1 mit R und Z = H und/oder Triazolo-epothilon B gemäß Formel 4b von Anspruch 1 mit R = CH₃ und Z = H mit einer Carbonsäure der Formel R'COOH unter Stickstoffabspaltung zu einem C-21-Ester von Epothilon A und/oder Epothilon B umsetzt und das (die) Produkt(e) der Umsetzung gewinnt 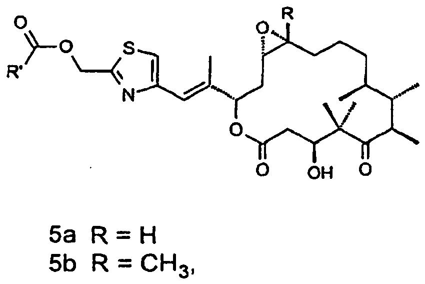 wobei R' bedeutet: H, Alkyl, Alkenyl, Alkinyl, Aryl oder Heteroaryl, wobei Alkyl, Aryl oder Heteroaryl eine Bedeutung gemäß Anspruch 1 besitzt.

9. Verfahren nach Anspruch 8, wobei in der Formel R'COOH der Rest R' C₂₋₈-Alkenyl oder C₂₋₈-Alkinyl bei jeweils beliebiger Lage der Mehrfachbindung bedeutet.

10. Photolytisches Verfahren, bei dem man Triazolo-epothilon A gemäß Formel 4a von Anspruch 1 mit R und Z = H oder Triazolo-epothilon B gemäß Formel 4b von Anspruch 1 mit R = CH₃ und Z = H in Gegenwart eines Nucleophilen NuH einer Photolyse unterwirft und als Produkt ein Epothilon A Analogon mit der Formel 5a bzw. ein Epothilon B Analogon mit der Formel 5b gewinnt

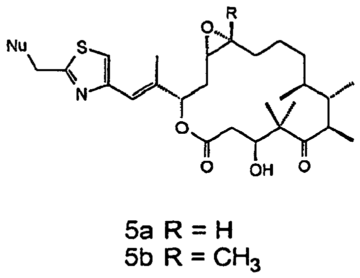

11. 1,3-dipolare Cycloaddition von Acetylendicarbonsäuredimethylester und Triazolo-epothilon A gemäß Formel 4a von Anspruch 1 mit R und Z=H oder Triazolo-epothilon B gemäß Formel 4b von Anspruch 1 mit R=CH₃ und Z=H zum Pyrazolderivat von Epothilon A mit der Formel 6a bzw. Epothilon B mit der Formel 6b

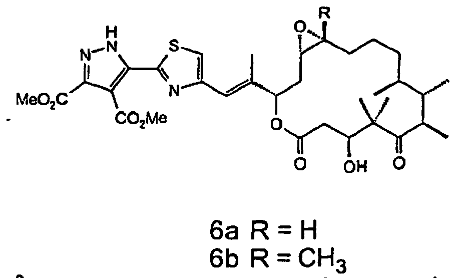

## Claims

1. Triazolo-thiazole analogues of epothilone A and epothilone B of formula 4a or 4b: 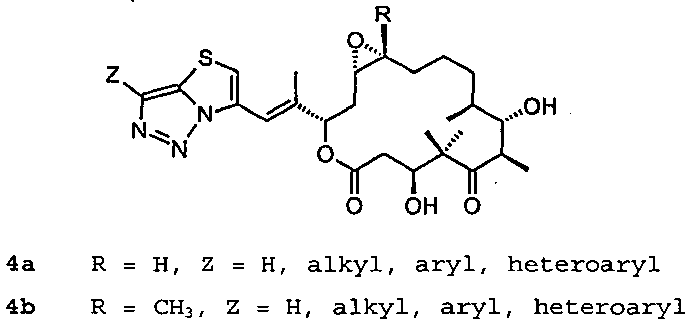 wherein:
R denotes H or CH₃
Z denotes H, alkyl, aryl or heteroaryl,
wherein
alkyl denotes methyl, ethyl, propyl, isopropyl, butyl, isobutyl, tert-butyl, pentyl or hexyl, optionally substituted by C₁-C₆alkoxy, C₁-C₆acyl, hydroxyl or halogen, especially Br, Cl, F or I, and/or
aryl denotes phenyl, o-, m-, p-tolyl, o-, m-, p-xylyl, benzyl, phenethyl or naphthyl, optionally substituted by C₁-C₆alkyl, C₁-C₆alkoxy, C₁-C₆acyl, hydroxyl or halogen, especially Br, Cl, F or I, and/or
heteroaryl denotes furyl, pyranyl, pyrrolyl, imidazolyl, pyrazolyl, pyridyl or indolyl, optionally substituted by C₁-C₆alkyl, C₁-C₆alkoxy, C₁-C₆acyl, hydroxyl or halogen, especially Br, Cl, F or I.

2. A method of preparing triazolo-thiazole analogues of epothilone A or epothilone B according to claim 1, wherein oxidative ring closure is performed on a hydrazone derivative of epothilone A or epothilone B of formula 3a or 3b: 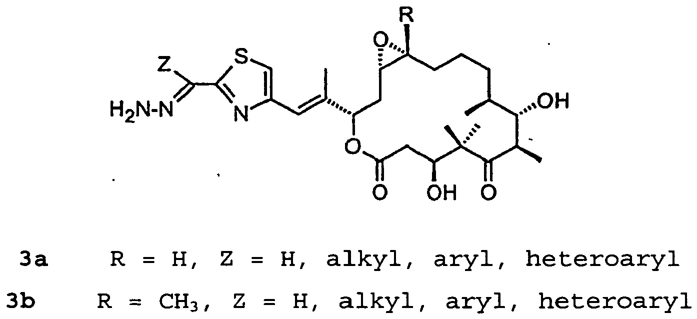 wherein R and Z are as defined hereinbefore, with the aid of metal oxides, K₃[Fe(CN)₆], lead tetraacetate or sodium hypochloride.

3. The method according to claim 2, wherein the metal oxide is NiO₂.

4. A pharmaceutical composition comprising or consisting of one or more triazolo-thiazole analogues of epothilone A or epothilone B according to claim 1 together with a pharmaceutically acceptable carrier, diluent or excipient.

5. The triazolo-thiazole analogue of epothilone A or epothilone B according to claim 1 or the pharmaceutical composition according to claim 4 for use in the treatment of tumour diseases and disorders of cell growth.

6. The triazolo-thiazole analogue of epothilone A or epothilone B according to claim 1 or the pharmaceutical composition according to claim 4 for use as in claim 5, wherein the tumour disease is cancer.

7. A fungicidal composition comprising or consisting of one or more triazolo-thiazole analogues of epothilone A or epothilone B according to claim 1 together with a carrier, diluent or excipient that is acceptable for fungicides.

8. A method of preparing C-21 esters of epothilone A of formula 5a and/or epothilone B of formula 5b, wherein triazolo-epothilone A according to formula 4a of claim 1 with R and Z = H and/or triazolo-epothilone B according to formula 4b of claim 1 with R = CH₃ and Z = H is/are reacted with a carboxylic acid of formula R' COOH with removal of nitrogen to form a C-21 ester of epothilone A and/or epothilone B and the product(s) of the reaction is/are recovered, 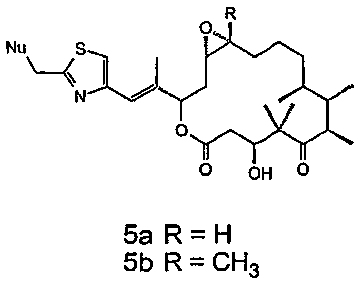 wherein R' denotes H, alkyl, alkenyl, alkynyl, aryl or heteroaryl, wherein alkyl, aryl or heteroaryl have the meaning according to claim 1.

9. The method according to claim 8, wherein in the formula R'COOH the radical R' denotes C₂-C₈alkenyl or C₂-C₈alkynyl, the multiple bond being in any desired position.

10. A photolytic method wherein triazolo-epothilone A according to formula 4a of claim 1 with R and Z = H or triazolo-epothilone B according to formula 4b of claim 1 with R = CH₃ and Z = H is subjected to photolysis in the presence of a nucleophile NuH and the resulting product is an epothilone A analogue of the formula 5a and an epothilone B analogue of the formula 5b, respectively

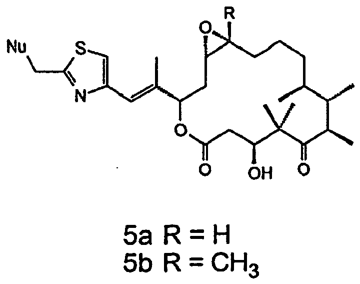

11. 1,3-Dipolar cycloaddition of acetylenedicarboxylic acid dimethylester and triazolo-epothilone A according to formula 4a of claim 1 with R and Z = H or triazolo-epothilone B according to formula 4b of claim 1 with R = CH₃ and Z = H to form a pyrazole derivative of epothilone A of formula 6a and epothilone B of formula 6b, respectively

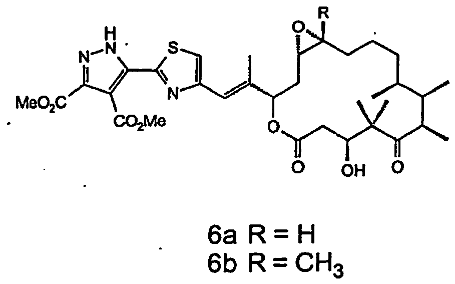

## Revendications

1. Analogues triazolo-thiazoles d'épothilone A et d'épothilone B comprenant la formule 4a ou 4b : 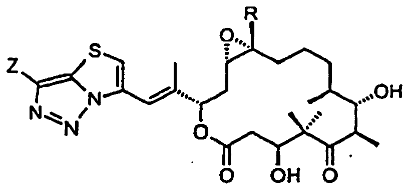 4a R = H, Z = H, un groupe alkyle, un groupe aryle, un groupe hétéroaryle
4b R = un groupe CH₃, Z = H, un groupe alkyle, un groupe aryle, un groupe hétéroaryle,
dans lesquels :
R signifie un atome H ou un groupe CH₃
Z signifie un atome H, un groupe alkyle, un groupe aryle ou un groupe hétéroaryle,
où :
un groupe alkyle signifie : un groupe méthyle, un groupe éthyle, un groupe propyle, un groupe iso-propyle, un groupe butyle, un groupe iso-butyle, un groupe tert.-butyle, un groupe pentyle ou un groupe hexyle, éventuellement substitué avec un groupe alcoxy en C₁-C₆, un groupe acyle en C₁-C₆, un groupe hydroxyle ou un groupe halogéné, en particulier de Br, de Cl, de F ou d'I, et/ou
le groupe aryle signifie: un groupe phényle, un groupe o-tolyle, un groupe m-tolyle, un groupe p-tolyle, un groupe o-xylyle, un groupe m-xylyle, un groupe p-xylyle, un groupe benzyle, un groupe phénétyle ou un groupe naphtyle, éventuellement substitué avec un groupe alkyle en C₁-C₆, un groupe alcoxy en C₁-C₆, un groupe hydroxyle ou un groupe halogéné, en particulier de Br, de Cl, de F ou d'I, et/ou
le groupe hétéroaryle signifie: un groupe furanyle, un groupe pyranyle, un groupe pyrrolyle, une groupe imidazolyle, un groupe pyrazolyle, un groupe pyridinyle ou un groupe indolyle, éventuellement substitué avec un groupe alkyle en C₁-C₆, un groupe alcoxy en C₁-C₆, un groupe hydroxyle ou un groupe halogéné, en particulier de Br, de Cl, de F ou d'I.

2. Procédé de fabrication d'analogues triazolo-thiazoles d'épothilone A ou d'épothilone B selon la revendication 1 dans lequel une cyclisation oxydative est entreprise sur un dérivé hydrazone d'épothilone A ou d'épothilone B avec la formule 3a ou 3b : 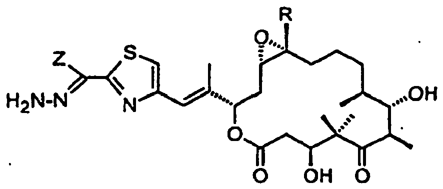 3a R = H, Z = H, un groupe alkyle, un groupe aryle, un groupe hétéroaryle
3b R = un groupe CH₃, Z = H, un groupe alkyle, un groupe aryle, un groupe hétéroaryle
R et Z ayant les significations définies ci-dessus, à l'aide d'oxydes métalliques, de K₃[Fe(CN)₆], de tétra acétate de plomb ou d'hypochlorure de sodium.

3. Procédé selon la revendication 2 où l'oxyde métallique est du NiO₂.

4. Composition pharmaceutique contenant ou constituée d'un ou de plusieurs analogues triazolo-thiazole d'épothilone A ou d'épothilone B selon la revendication 1, à côté d'un support, d'un diluant ou d'un produit auxiliaire pharmaceutiquement acceptable.

5. Analogue triazolo-thiazole d'épothilone A ou d'épothylone B selon la revendication 1 ou composition pharmaceutique selon la revendication 4 pour une utilisation lors du traitement de maladies tumorales et de troubles de la croissance cellulaire.

6. Analogue triazolo-thiazole d'épothilone A ou d'épothilone B selon la revendication 1 ou composition pharmaceutique selon la revendication 4 pour une utilisation telle qu'en revendication 5 où il s'agit du cancer dans le cas de la maladie tumorale.

7. Composition fongicide contenant ou constituée par un ou plusieurs analogues triazolo-thiazole d'épothilone A ou d'épothilone B selon la revendication 1 à côté d'un support, d'un diluant ou d'un agent auxiliaire acceptable pour des fongicides.

8. Procédé de fabrication d'esters C-21 d'épothilone A possédant la formule 5a et/ou d'esters C-21 d'épothilone B possédant la formule 5b, dans lequel on fait réagir de la thiazolo-épothilone A selon la formule 4a de la revendication 1 avec R et Z = H, et/ou de la triazolo-épothilone B selon la formule 4b de la revendication 1 avec R = le groupe CH₃ et Z = un atome d'H, avec un acide carboxylique de formule R'COOH, moyennant une élimination d'azote, en un ester C-21 d'épothilone A et/ou d'épothilone B et on recueille le (les) produit(s) de la réaction 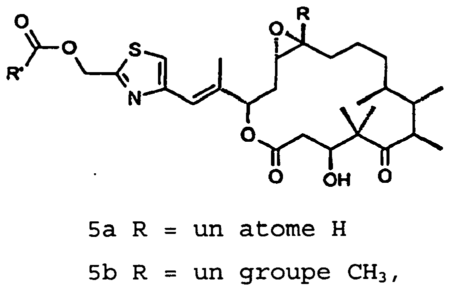 où R' signifie : un atome d'H, un groupe alkyle, un groupe alcényle, un groupe alcinyle, un groupe aryle ou un groupe hétéroaryle, où les groupes alkyle, aryle ou hétéroaryle possèdent une signification selon la revendication 1.

9. Procédé selon la revendication 8 où, dans la formule R'COOH, le radical R' représente un groupe alcényle en C₂₋₈ ou un groupe alcinyle en C₂₋₈ dans n'importe quelle position de la liaison multiple.

10. Procédé photolytique dans lequel on soumet du triazolo-épothilone A selon la formule 4a de la revendication 1 avec R et Z = H, ou du triazolo-épothilone B selon la formule 4b de la revendication 1 avec R = un groupe CH₃ et Z = un atome d'H, à une photolyse en présence d'un nucléophile NuH et on obtient en tant que produit un analogue d'épothilone A de la formule 5a, respectivement un analogue d'épothilone B de la formule 5b

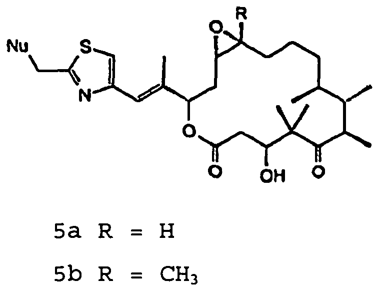

11. Cycloaddition 1,3-dipolaire de l'ester diméthylique de l'acide acétylène dicarboxylique et du triazolo-épothilone A selon la formule 4a de la revendication 1 avec R et Z = H ou de triazolo-épothilone B selon la formule 4b de la revendication 1 avec R = un groupe CH₃ et Z = un atome d'H, pour former un dérivé pyrazole d'épothilone A de la formule 6a, respectivement un dérivé pyrazole d'épothilone B de formule 6b
